# EUROPEAN PATENT APPLICATION

(11) **EP 3 187 936 A2**
(43) Date of publication of application: **05.07.2017**
(21) Application number: 14823290.3
(22) Date of filing: 29.08.2014
(51) Int. Cl.: G03B 21/60, G06T 17/00, G03B 21/10, G03B 21/56

(54) **DEVICE FOR DISPLAYING IMAGES ON SURFACES OF ANATOMICAL MODELS AND CORRESPONDING METHOD**

(71) Applicant: Ortigoza Sequeiros, Carlos, 53950 Naucalpan de Juárez (MX)
(72) Inventor: Ortigoza Sequeiros, Carlos, 53950 Naucalpan de Juárez (MX)
(74) Representative: Pons Ariño, Angel
(86) International application number: PCT/IB2014/064155
(87) International publication number: WO 2015/004647

(57) **Abstract**

The invention relates to a device for displaying images on surfaces of anatomical models, and a method for projecting images onto to-scale anatomical models, allowing medical professionals and patients to observe the effects of a particular surgical procedure, or the effects of the passage of time, on a part of the body. In addition, the invention can be used in the field of forensic and anthropological reconstruction for the recognition of people or living beings without them being present.

## Description

### Field of invention

The present invention refers to a device for displaying images on surfaces of anatomical models and to a method for projection of images on scale anatomical models which allows professional of medicine and patients to visualize the effects resulted from a particular surgical procedure or with the passage of time over a part of the body.

### Background and state of the art

One of the main problems that undergo professional of medicine during the issue of professional opinions for a medical treatment or surgical procedure is the effectiveness in transmitting their ideas to patients, as well as the delivery of expectations of a patient to a medical. For example, when a patient visits a plastic surgeon, master in aesthetic and/or reconstructive surgery, who is the professional specialized in medical aesthetic and reconstructive surgery, the surgeon requires to explain the patient which will be the procedure needed to be carry out to meet the needs of the patient and which are the differences with the expected result from the actual procedure. Following the given example, plastic surgeons and cosmetic surgery masters's perform aesthetic and reconstructive surgeries alike, for improving quality life of patient, whereby only if the patient feels latch with the modifications that his body will suffer, he will give his consent to the procedure. In a septoplasty, surgeon must explain the patient the form in which he will approach the nasal septum, thus potentially it can change appearance of the patients face. Other medical professionals have similar problems with transmitting their ideas to their patients, such as the maxillofacial surgeons, orthopedist, kinanthropometry, forensic experts, and veterinary surgeons, among others, whereby the device claimed herein can serve to all aforementioned medical specialities and other which find it useful in similar situations.

In the event of such challenges, health professionals have turned to various methods in order to explain in the best possible way the result expected to obtain from a treatment or specific method. Traditionally, surgeons turned to display photographs of previous treated patients which had the same or similar affliction. However, this solution has serious problems. One of these problems are [sic] possible breaches to confidentiality obligations between patient and doctor. Another problem, may be that medical professionals have not treated identical or similar afflictions, therefore, they have no pictures or images for display to their patients. Furthermore, every patient features and clinical responses to medical procedures are different from each patient, whereby if it is possible to create a general idea of the results expected to obtain from medical interventions, it is never possible to have certainty over final results.

On account of the problems presented by displaying pictures or images of the above medical procedures, the health professionals have turned to other methods. One of these methods is manipulating digital images by computer, as the predicted by the patents of the United States of America numbers US 8,620,038B2 *Method, system and computer program product for automatic and semi-automatic modification of digital images of faces* and US 6,502,583 B1 *Method of correcting face image, makeup simulation method, makeup method makeup supporting device and foundation transfer film.* With the methods in aforementioned, as well as with other devices and methods that are currently in the state of the art, the professional of medicine take photographs of their patients before performing medical or surgical procedures and manipulate the images to simulate the results expected from those procedures. The problem faced by health professionals whom carry out this type of simulations is that they cannot display to patients a full view of the anatomical member which will be affected by the procedure, thus being limited to the view in two dimensions that can be displayed on the screen of the computer.

To overlap the limitation in two dimensions imposed by the screen of a computer, health professionals have turned to simulations in three dimensions. An example of this type of modifications is provided by United States of America patent number US 8,026,916B2 entitled *Image-based viewing system.* For the creation of tridimensional scenes by computer, it is conventionally used a coordinate system (cartesian plane). Thus the axes of the height, length and depth are defined, and intermittent equidistant values are assigned, so that a specific point can be located within the coordinate system. Thus, when a draw of a geometrical body is needed within the coordinate system, as a pyramid may be, for example, the coordinates of the vertexes are determined. The triangular surfaces are normally used because they are the only figure always coplanar., thereby preventing deformity problems of the images generated by the computer. To represent the faces of the object it is fixed in three coordinates: one for each vertex of each object's face. Then a system is determined by which the order of coordinates appointment indicates whether it is the front or rear of each face. A transformation, as the scaling, translation and rotation can be applied to the geometric bodies already drawn by the computer, then. When the three dimensional scene is already drawn, the computer must convert it to two dimensional in order to be able to project it in the pixels of the computer screen. In this way, all of the effort taken to create a three dimensional scene is diminished as in the screen it is displayed as a two dimensional scene on the screen. It is true that many implemented computer methods enable the scene to be drawn in real time to provide the illusion of being three dimensional, so that the image can be rotated and viewed from different angles. However, the patient only has the illusion of three dimensional viewing an image, when actually he can only see two dimensional images as they are projected by the pixels of the screen. As well, if the patient is not facing the screen of the computer precisely, the image will be distorted even more in accordance with the angle of his field of vision in connection with the screen.

This leds to additional problems that may have as a result that the patient visualizes itself with a results's projection of the medical intervention radically different from the final result.

To avoid these problems, a new device is hereby proposed for displaying images on surfaces of anatomical models and a method for the correct visualization of changes that the patient will suffer following a medical intervention.

The device object of the invention and the associated method can also be used for forensic experts and anthropologist. An example of use to the invention here claimed by a forensic expert is while identifying a person, on which a digital image of the person to identify is superimposed projected onto the corresponding anatomical model. Furthermore, a portrait spoken artist can use a digital projection of the drawing created on an anatomical model disposed in the a device claimed herein, to give greater depth and relief to the person's features, thus helping his early identification. Anthropologists can use similar techniques to provide higher life to the persons they want to identify after being uncovered during professional activities among experts in anthropology and history.

### Object of the invention.

The object of the invention is a device and associated method suitable for professional of human or animal health, to show their patients or clients the results of a certain medical or surgical intervention in the patient's body, in a three dimensional, interactive manner, and in the least possible time. In addition, it lowers the operating costs in comparison with other solutions to problems like, eliminates the need for acquiring professional software licenses for manipulation of images in two and three dimensions, and can be transported to other locations for mobile services.

Likewise, anthropologist and forensic experts can benefit from the use of the device and the method claimed hereby from better identification of persons and human remains.

### Description of the drawings.

Preferred embodiments of the aforementioned invention will be described below as an example only with reference to the figures added.
Figure 1 is a front view of the display device to display images on surfaces of anatomical models in the preferred embodiment. The device for display anatomical models 100 is at the bottom. A device for digital projection 200 is opposite thereto, projecting a digital image onto the anatomical model 130. The optional support for alignment 300 holds the display device of anatomical models 100 in perfect perpendicular alignment to the digital projection device 200.
Figure 2 is a diagonal view of the display device of anatomical models 100 with the digital projection device 200 in operation.

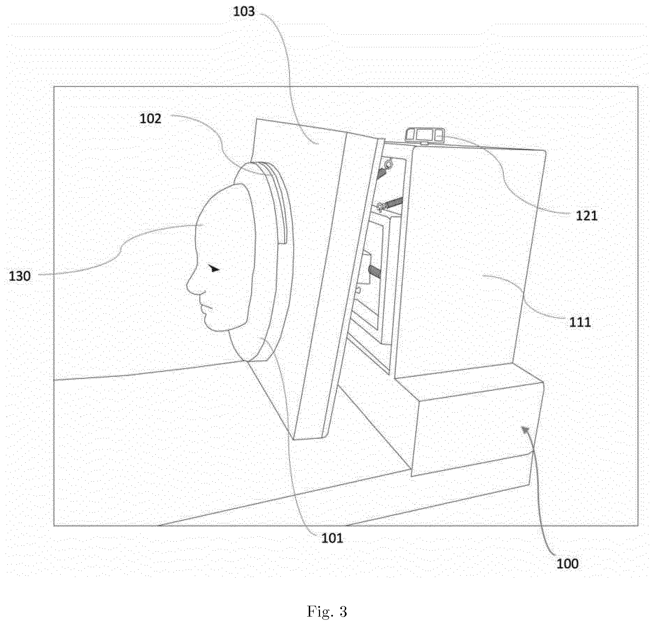

Figure 3 is a side view of the display device of anatomical models 100 claimed.

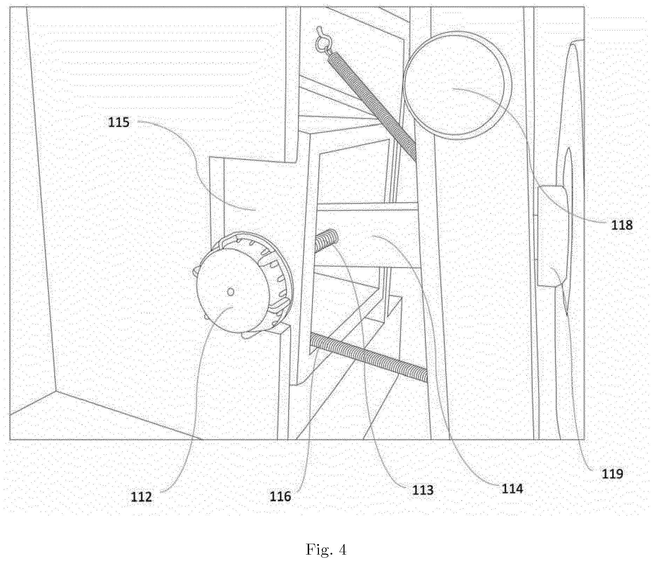

Figure 4 is a view of the inside mechanism of the display device of anatomical models 100.

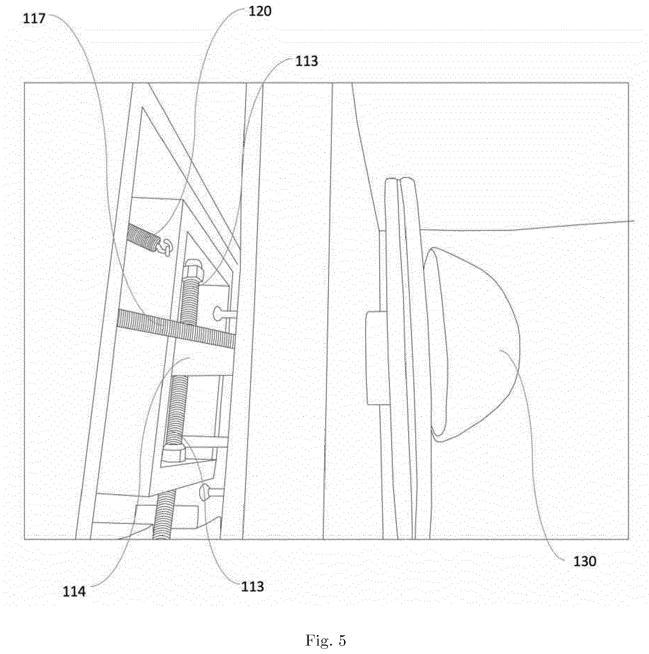

Figure 5 is a top view of the device model anatomical models 100 and its corresponding inside mechanism.

### Detailed description of the claimed invention.

The patterns represent the preferred embodiment of the device claimed.

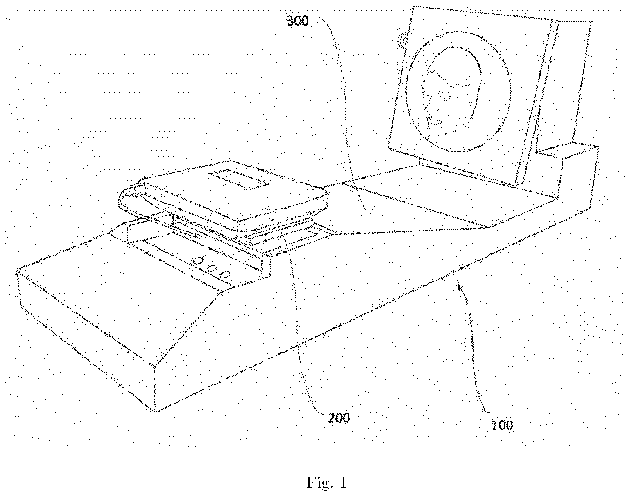

Figure 1 illustrates the display device of anatomical models 100. In front of the display device of anatomical models 100 is a digital projection device 200. In a mode, an optional support for alignment 300 can be used to align the display device of anatomical models 100 with a digital projection device 200.

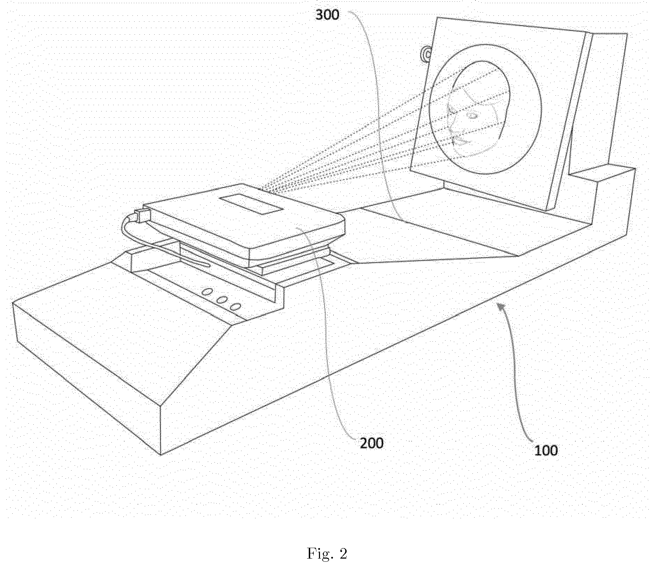

The digital projection device 200 is used to continuously and permanently project an image on an anatomical model 130, as seen in Figure 2. In one embodiment, said anatomical model 130 may be the generic model of a human or animal part. In another embodiment, an anatomical model 130 is prepared with concrete specifications to meet the needs of a patient or a client.

The anatomical model 130 is fixed to a support and placing plate of anatomical model 101. In one embodiment, said anatomical model 130 is fixed to the support and placing plate of anatomical model 101 by means of any type of adhesive or cement. In another embodiment, the anatomical model 130 is fixed to the support and placing plate of anatomical model 101, by means of magnets. In yet another embodiment, the anatomical model 130 is fixed to the support and placing plate of anatomical model 101 by means of screws or nails.

The support and placing plate of anatomical model 101 is disposed in a radial adjustment plate 102, which is fixed to prevent unwanted ways of movements. The plate rotates three hundred sixty degrees on the rotary joint 119 to allow the anatomical model 130 has any desired orientation in perpendicular plane to the projection generated by the digital projection device 200. The rotary joint. 119 attach the radial adjustment plate 102 with the perpendicular inclination plate 103. Said perpendicular inclination plate 103 is attached by a hinge to the support arm 114. By means of a spindle, the radial adjustment knob 118 allows the perpendicular inclination plate 103 adapts in a fixed angle. In the preferred embodiment, the angle of inclination that can be achieved by the perpendicular inclination plate 103 with respect to the vertical is forty five degrees, measured from the upper or lower end, taking into account that the movement axis is the hinge joint with the support arm 114. To avoid unwanted movements of the perpendicular inclination plate 103 caused by the angular momentum generated by the weight of said plate against the hinge joint of the support arm 114 the resistors 116 and 117 are added, giving thus precision to the location of the anatomical model 130 for the projection of digital image.

The support arm 114 is attached to the movement compartment 115 at two points of contact. The first contact point is located at the distal end of the support arm 114. In one embodiment, the distal end of the support arm 114 is connected by a guide bar parallel to the resting surface of the display device of anatomical models 100, so that it prevents the angular momentum of said support arm 114 caused by the weight of the perpendicular inclination plate 103 and the bearing point on the lateral movement spindle 113. The second point of contact is by means of a lateral movement spindle 113, which pierces one of the walls of the movement compartment 115 and extends outside the main support structure 111. The lateral adjustment knob 112 is attached to the lateral movement spindle 113 on its extended end, so that the user can rotate it to modify the position in respect of the vertical central middle of the support arm 114 and, therefore the perpendicular inclination plate 103 and the anatomical model 130.

The movement compartment 115 is supported by the vertical movement spindle, which is extended at the upper lid of the main support structure 111 to abut on the vertical adjustment knob 121. By the rotation of the vertical adjustment knob the vertical movement spindle is rotated, raising or lowering the movement compartment 115 relative to the plane of rest of the display device of anatomical models 100. Accordingly, the user has the ability to adjust the position of the anatomical model 130 for adjusting the image projected on it by the digital projection device 200.

Having mounted the disclosed device at the location that will be used, the user will need placing the digital projection device 200 in front of the display device of anatomical models 100. The projector lens of the digital projection device 200 will have to be facing the anatomical model 130. In the preferred embodiment, the line of sight between the projection lens of the digital projection device 200 is positioned at an angle of ninety degrees facing the anatomical model 130, so that the image projected on the anatomical model is adjusted.

The user of the device must prepare the anatomical model 130 which will be placed on the display device of anatomical models 100. In one embodiment, said anatomical model 130 is created with a moldable material, so that a user can define manually changes for displaying on the anatomical element. For example, a user surgeon can be used plastic materials such as plaster, clay, or sealants for creating a model of the patient's face on which to modify certain features to show as desired so after the medical procedure and modificarlo according to the specifications of the patient. In another embodiment, the anatomical model 130 is created by molding methods for solid non modifiable. For example, a forensic expert user may make a model anatomical 130 plastic by thermoforming, so as to have the shape of the face of a person disappeared. The user of the device deberá then preparing an image for proyectarla on the anatomical model 130. In one embodiment, the user takes a picture of the patient's anatomical element that is modificará. For example, a surgeon plastic may take a picture of the front face of the patient to be projected onto the anatomical model 130, so that the facciones and characteristics of the patient is reflected in the model anatomical 130. In another embodiment, the user can prepare an image in which the demuestre characteristics that normally would be hidden. For example, a forensic expert user can prepare an image on which to view the central nervous system of an anatomical element that is being projected onto the anatomical model 130, for graphically demonstrate any injury. However, in all cases the image to the user brew deberá being specularly symmetrical to the anatomical model 130, so that the image is projected on said anatomical model 130.

## Claims

1. A device for displaying images on surfaces of anatomical models comprising;
An anatomical model to scale;
A mechanism to align the appropriate anatomical model in the vertical, horizontal, depth, and perpendicular rotation axis;
A digital image projector.

2. The device of claim 1, in which the suitable anatomical model is mounted to the general support and display structure in a removable manner.

3. The device of claims 1 and 2, in which the anatomical model to scale is designed according to corresponding anatomical modification expectations after a medical or surgical procedure.

4. The device of claims 1 and 2, in which the anatomical model to scale is designed according to criteria of forensic or anthropologic reconstruction.

5. A method for producing and projecting images, comprising:
An image of an anatomical element;
The digital projection of the anatomical element image on an anatomical model to scale.

6. The method described in claim 5, in which the anatomical element image is designed according to the characteristics of a given clinical patient.

7. The method described in claim number 5, in which the anatomical element image is designed with forensic or anthropologic reconstruction criteria.

8. The method described in claims 5, 6, and 7, in which the image has mirror-image symmetry from the anatomical model on which it will be projected.
